# EUROPEAN PATENT SPECIFICATION

(11) **EP 1 702 632 B1**
(45) Date of publication and mention of the grant of the patent: **31.10.2012**
(21) Application number: 04819977.2
(22) Date of filing: 03.12.2004
(51) Int. Cl.: A61L 27/36, A61L 27/38, A61F 2/10

(54) **HAIR GROWTH METHOD**
HAARWUCHSMETHODE
PROCEDE DE POUSSE DE CHEVEUX

(30) Priority: 05.12.2003 JP 2003408220
(43) Date of publication of application: 20.09.2006
(73) Proprietor: BIOINTEGRENCE INC., Hiroshima-shi, Hiroshima 733-0013 (JP); Phoenixbio Co., Ltd., Higashihiroshima-shi, Hiroshima 739-0046 (JP)
(72) Inventor: YOSHIZATO, Katsutoshi, Higashihiroshima-shi,Hiroshima (JP); SHIMADA, Takashi, Higashihiroshima-shi, Hiroshima (JP); TOYOSHIMA, Koei, Higashihiroshima-shi, Hiroshima (JP); MATSUNAGA, Mikaru, Higashihiroshima-shi, Hiroshima (JP)
(74) Representative: Leathley, Anna Elisabeth
(86) International application number: PCT/JP2004/018421
(87) International publication number: WO 2005/053763

(56) References cited:
- WO-A-99/01034
- WO-A-03/068248
- JP-A- 10 118 103
- JP-A- 11 180 878
- JP-A- 2000 187 030
- JP-A- 2002 145 701
- JP-A- 2003 070 466
- JP-A- 2003 189 849
- JP-A- 2003 238 421
- REYNOLDS ET AL: "Hair follicle reconstruction in vitro" JOURNAL OF DERMATOLOGICAL SCIENCE, ELSEVIER SCIENCE PUBLISHERS, SHANNON, IR, vol. 7, 1 July 1994 (1994-07-01), pages S84-S97, XP022501993 ISSN: 0923-1811
- MATSUZAKI T ET AL: "Role of hair papilla cells on induction and regeneration processes of hair follicles." WOUND REPAIR AND REGENERATION : OFFICIAL PUBLICATION OF THE WOUND HEALING SOCIETY [AND] THE EUROPEAN TISSUE REPAIR SOCIETY 1998 NOV-DEC, vol. 6, no. 6, November 1998 (1998-11), pages 524-530, XP002543951 ISSN: 1067-1927

## Description

### Technical Field

The invention of this application relates to a hair growth method by transplantation of dermal papilla cells and to a transplanting material used for the method.

### Background Art

Hair follicles producing the hair shafts are induced by an interaction between special mesenchymal cells, dermal papilla cells and epidermal cells. It has been believed that dermal papillae deeply participate in the regulation of the hair cycle, which is a repeated cycle of hair follicle development, producing and elongation of hair shafts, and involution of hair follicles. When the hair cycle becomes irregular by various causes such as a decrease of blood flow rate in hair bulbs and an increase of androgen concentration, male pattern baldness (androgenetic alopecia) appears. At the later stage of androgenetic alopecia, the effect of hair restorers is restricted and, in addition, density of hair follicles also becomes sparse whereby there has been demanded a therapeutic art for increasing the number of hair follicle tissue by cell transplantation.

In the recent therapeutic art, it has been reported that a method of auto-transplantation of healthy scalp skin with hair follicles from occipital area to frontal and/or sincipital scalp with alopecia was developed to reduce the area of the alopecic site (Non-Patent Document 1). A clinical method in which hair follicular units were surgically separated from healthy scalp and grafted into the alopecic site has been also reported and a therapeutic effect is available (Non-Patent Document 2). In any of those methods however, although normal hairs were able to be grown in the alopecic site, it is still unavoidable that total numbers of healthy hair follicles or hair shafts are the same as before or even reduced. That is because only healthy follicles are just moved to the alopecic site. Accordingly, when alopecia within a broad range is to be treated, it is necessary that normal scalp of broader range is subjected to a skin flap formation or, after the excision, donor tissue to supply normal scalp skin with hair follicles. Therefore, it is necessary that a mechanical stretcher is previously inserted into a hypodermic tissue under the normal scalp skin to be utilized and the skin is gradually expanded (Non-Patent Document 3) or the operation is divided into several times to wait for the expansion of the normal scalp skin. Thus, in spite of the auto-transplantation, the current status is that the supply of normal scalp donor tissue is not enough as in the case of organ transplantation such as liver and heart transplantation. In addition, very high surgical technique and separation of scalp tissues and hair follicles by handwork are demanded.

Such a surgical method is very highly invasive and causes very much pain and burden to patients. On the other hand however, that is effective as a fundamental therapeutic method only where the site in which alopecia appears is covered by follicles in a healthy state and normal hairs which are kept can be prepared even if exposed to various causes of alopecia.

In view of the above, there has been a demand for artificial hair-follicles constructed from culture cells for transplantation where pain and burden of the alopecic patients are mitigated, short supply of donors of normal scalp tissues with healthy hair follicles is solved and high transplantation technique is not necessary. With regard to artificial hair follicles for transplantation, a method has been carried out where a biodegradable and biocompatible polymer is inserted into the skin and is made to be compatible to dermal tissue and subcutaneous tissue. However, in such a method, considerable immune rejections and infectious diseases are generated and the method has been prohibited in the United States already.

Under such circumstances, there will be a method where the cells from a patient himself/herself are separated from very small healthy tissue, proliferation by cell-cultivation techniques and constituted into germ of hair follicles using them as a material whereby hair follicles for transplantation are increased. Since hair follicles are formed from the cultured propagated cells of a patient himself/herself, no immune rejection occurs and, in addition, since very high biocompatibility is available, no foreign body response happens as well whereby repair of the transplanted tissues finishes quickly. Accordingly, unlike the transplantation of artificial hair, it is not necessary to involve a high risk of infectious diseases for a patient during the several days to several weeks until the surroundings of the artificial hairs become epithelium. For example, in Patent Document 1, a method is disclosed wherein hair dermal papilla cells isolated from a patient himself/herself are grown by incubation and the resulting cultured hair dermal papilla cells are transplanted to a patient. However, even when the hair dermal papilla cells which are grown by incubation are transplanted to the skin, efficiency for hair growth is very low, and even if hair is grown, the state of the hair is weak and the actual status is that such a thing is hardly said to be regeneration of natural hair.

For artificial induction of the hair follicles, it is necessary that an embryological finding about hair follicle development and a means of tissue engineering are applied. As mentioned above, hair follicles are embryologically developed by an interaction of epidermal cells with mesenchymal cells, especially called dermal condensation. It has been reported that the interaction for the development of hair follicles as such is able to be reproduced by an experiment where cultured dermal cells and epidermal cells derived from newborn rat are mixed and transplanted into the back of immune deficiency mouse (Non-Patent Document 4). It has been also reported that a method for the regeneration of follicular bulb is carried out by transplantation of human or animal dermal papilla with animal hair follicle that removed follicular bulb (Non-Patent Document 5). Accordingly, it has been already known to be able to be applied to the treatment of human alopecia.

It has been also reported that the intact dermal papillae freshly isolated from hair bulbs or artificial dermal papillae comprising cultured dermal papilla cells are transplanted by hand into a space between dermis and epidermis by incising the skin with a pair of sharp forceps or knife whereby new hair follicles are able to be induced from interfollicular epidermis (Non-Patent Document 6). A method where allogenetic transplantation is carried out to the skin of rat ear using a syringe and needle has been also attempted and, although the result is limited, it has been reported that hair follicle structures are induced and hair shafts were produced and elongated by these hair follicles to intact dermal papillae or the site to which cultured dermal papilla cells are transplanted (Patent Document 1). However, in the case of formation of hair follicles by an interaction of epidermal cells with dermal papilla cells, it is necessary that they are made quite close to each other until the interaction of epidermal cells with dermal papilla cells is able to take place. With regard to a method by which the above is able to be achieved, there may be a method where dermal papilla cells are transplanted into the area which is just under the epidermal cells.

In conducting a transplantation of the dermal papilla cells into the position that is quite close the place just under the epidermal layer, a high technique where the position for transplanting the cells without doing so diffusely is controlled with very high precision is required. In the dermal layer, fibers of extracellular matrix mainly comprising collagen constructed by fibroblasts are complicatedly entangled. When a carrier for a drug transportation such as microtissues, cells and collagen beads is injected into the inner part of the skin, it is inevitable to inject after suspending in a physiological saline solution, medium, serum or the like. In that case, the interstitial fibers are cleaved by the pressure upon injection. Therefore, in the injection of intact dermal papillae, artificial dermal papillae, and any types of cells etc. using a syringe, it is very difficult to transplant and localize to the place immediately under the epidermis where the interaction between dermal and mesenchymal cells can be expected. Accordingly, if that is able to be achieved in terms of the technique, quite a lot of time is needed for conducting the transplantation of several thousand to several tens of thousands of cells and, as a result, that is added to the cost for development and to the cost for treatment.

In a method where epidermis is incised by tweezers and knife and transplanted just under the epidermis by handwork, there are required skill and labor as well as a lot of damage to the recipient skin whereby it is substantially impossible to induce several thousand to several tens of thousands of hairs which are needed by a patient suffering from alopecia.

In the meanwhile, the inventors of this application clarified by transplantation of subcultured dermal papilla cells into the spaces between epidermis and dermis of rat soles. Furthermore dermal papilla cells for transplantation isolated from rat vibrissae are able to be subcultured for a long period when supernatant of conditioned medium of primary cultivation of epidermal cells of rat sole skins or fibroblast growth factor 2 (FGF 2) and that the subcultured dermal papilla cells retain a hair follicle inducing ability throughout the subcultures for several tens of passages and have filed a patent application already (Patent Document 2). The inventors of this application have also invented a method where a predetermined amount of fine organism material such as dermal papilla cells is discharged from a discharging device such as a syringe in a sure and stable manner so as to transplant to the skin and have filed a patent application already (Patent Document 3).

As mentioned hereinabove, it is necessary for induction of the hair follicles by transplantation of dermal papilla cells that intact dermal papillae or cultured dermal papilla cells are surely made to be near the epidermal layer and much more of them are transplanted where burden to the recipient is made small and that efficient hair growth results from the transplanted dermal papilla cells.

Incidentally, it has been also known that dermal papilla cells decrease their ability for induction of hair-follicle regeneration and hair -shaft growth by repetition of subcultures for about ten passages (Non-Patent Document 7). With regard to that, the inventors of this application have invented an incubating art where dermal papilla cells are grown under such a state that the ability for induction of hair follicles is able to be retained for a long period (Patent Document 3). However, even in the case of the dermal papilla cells that are cultured and grown by the method of the Patent Document 3, although regeneration of hair follicles after the transplantation is possible, the situation that the ability of induction of hair growth decreases together with subculture and is lost is still the same. Accordingly, there has been a brisk demand for a method where transplantation is carried out under such a state that a sufficient ability for hair growth is applied to the dermal papilla cells that are propagated by subcultures for a long period.

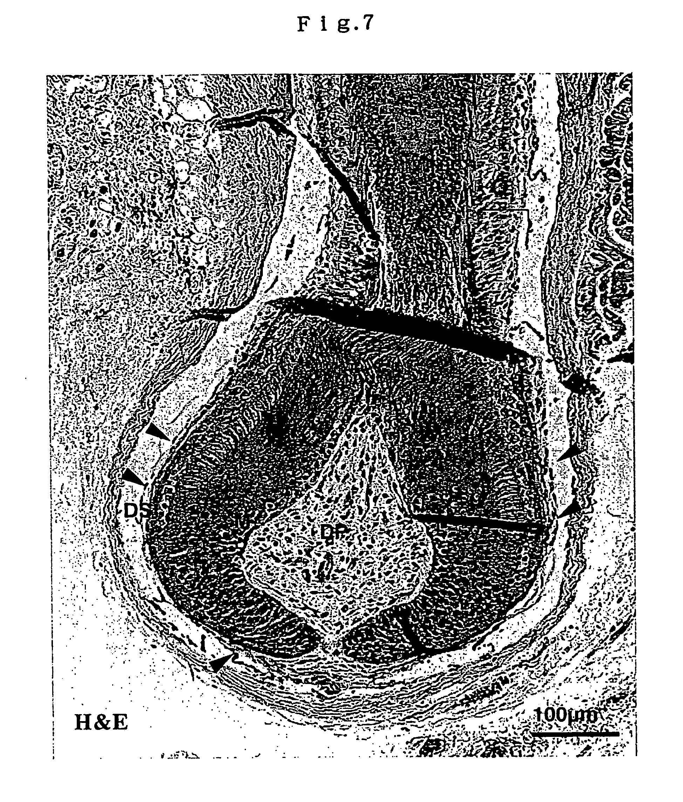

On the other hand, dermal sheath is a tissue comprising dermal cells surrounding the outermost layer from end of bulb to bulge region of hair follicle. The dermal sheath connects to dermal papilla at the lowest end of hair bulb (arrow heads in Fig. 7). Incidentally, when hair follicles wherefrom dermal papilla and hair matrix are partially excised are transplanted in renal capsule or hypodermic tissue, the hair bulb part is regenerated and elongation of hair shaft is observed. Accordingly, it has been believed that precursor cells of hair dermal papilla cells are distributed in dermal sheath.

It has been recently found that, when an experiment where dermal sheath freshly isolated from male scalp is trans-gender grafted to female forearm skin, formation of hair follicle is induced and hair-shaft is outgrown from surface of recipient skin (Non-Patent Document 8). It has been also reported that, when primarily cultured dermal sheath cells are transplanted to the sites which is just under the interfollicular epidermis of the rat ear, formation of hair follicle is induced and hair is grown (Non-Patent Document 9). From those facts, it has been believed that dermal hair root sheath cells are also a hopeful transplantation material for regeneration of the hair.

However, in the case of dermal sheath cells, although ability for induction of hair follicles and growth of hair is also noted, no significant hair growth is noted in dermal sheath cells that are grown by subculture (Non-Patent Document 9).

Non-Patent Document 10 discloses reconstruction of hair follicles in *vitro*, using dermal papilla, dermal sheath and germinative epidermal cells in combination with basal outer root sheath cells grown on a hair follicle glass membrane from rat. Although this demonstrates the production of hair follicles, no true hair growth is shown.

Patent document 4 discloses a method of inducing new hair growth in humans comprising implanting human dermal papilla cells into human scalp skin. Papilla cells are cultured in a medium which may be supplemented with known papilla growth factors such as fibroblast, vascular endothelial or platelet-derived growth factors and/or other known papilla stimulators. Cells of high passage number may be used to maximize cell number from a single papilla.

Patent document 5 discloses a method for inducing hair follicle formation comprising delivering inductive dermal sheath cells and/or dermal papilla cells directly into the dermal layer using a controlled deliver device.

Patent Documents:
1. Japanese Patent Laid-Open No. 2001/302,520
2. Japanese Patent Laid-Open No. 07/274,950
3. Japanese Patent Laid-Open No. 2003/235,990
4. WO 99/01034 A (Cooley, Jerry E. [US]; Vogel, James E. [US], 14 January 1999)
5. WO 03/068248 A (INTERCYTEX LIMITED [GB) 21 August 2003)

Non-Patent Documents:
1. Stough DB et al., Surgical procedures for the treatment of baldness, Cutis., 37(5), 362-5, 1986
2. David Julian et al., Maicrograft size and subsequent survival, Dermatol. Surg., 23, 757-762, 1997
3. Wieslander JB, Repeated tissue expansion in reconstruction of a huge combined scalp-forehead avulsion injury, Ann Plast Surg., 20(4), 381-5, 1988
4. Lichti U et al., In vivo regulation of murine hair growth; insights from grafting defined cell populations onto nude mice, J Invent Dermatol., 101(I) 1245-129S, 1993
5. Jahoda CA et al., Trans-species hair growth induction by human hair follicle dermal papillae, Exp Dermatol., 10(4):229-37, 2001
6. Jahoda CA, Induction of follicle formation and hair growth by vibrissa dermal papillae implanted into rat ear wounds: vibrissa-type fibres are specified. Development., 115(4), 1103-9, 1992
7. Weinberg WC et al., Reconstitution of hair follicle development in vivo: determination of follicle formation, hair growth, and hair quality by dermal cells. J. invest. Dermatol., 100, 229-236, 1993
8. Reynolds AJ et al., Trans-gender induction of hair follicles., Nature, 402, 33-34, 1999
9. McElwee KJ et al., Cultured peribulbar dermal sheath cells can induce hair follicle development and contribute to the dermal sheath and dermal papilla. J. invest. Dermatol., 121, 1267-1275, 2003
10. Reynolds AJ et al., Hair follicle reconstruction in vitro, Journal of Dermatological Science, vol. 7, S84-S97, 1994.

### Disclosure of the Invention

An object of the invention of this application is to provide a method where, in regeneration of hair by transplantation of dermal papillae or cultured dermal papilla cells to the skin, a highly efficient hair growth is resulted and, further, the hair growth is induced to the state near the natural hair and also to provide a material for the transplantation therefor. As a means for solving the above, the following invention is provided.

In a first embodiment, the present invention includes a composition for use in inducing hair growth, containing
(a) human dermal papilla cells;
(b) human epidermal cells; and
(c) human dermal sheath cells,
wherein the cells (a), (b) and (c) are single cells.

In said composition, the components may be derived from human scalp.

Alternatively or in addition, the dermal papilla cells of component (a) above may be cultured cells.

Alternatively or in addition, the dermal papilla cells of the component (a) may be cells subcultured for 10 or more passages and the component (c) may be dermal sheath cells of the hair bulb.

Alternatively or in addition, the dermal sheath cells of the component (c) may be cultured cells.

Alternatively or in addition, the dermal sheath cells of the component (c) may be cultured cells which are grown in a medium containing FGF2.

In a further embodiment, the present invention includes use of a composition containing
(a) human dermal papilla cells;
(b) human epidermal cells; and
(c) human dermal sheath cells,
in the manufacture of an agent for use in inducing hair growth, wherein the cells (a), (b) and (c) are single cells.

In said use, the components may be derived from human scalp.

Alternatively or in addition, the dermal papilla cells of the component (a) may be cultured cells.

Alternatively or in addition, the dermal papilla cells of the component (a) may be cells subcultured for 10 or more passages and the component (c) is dermal sheath cells of the hair bulb.

Alternatively or in addition, the dermal sheath cells of the component (c) may be cultured cells.

Alternatively or in addition, the dermal sheath cells of the component (c) may be cultured cells which are grown in a medium containing FGF2.

In addition disclosed herein is a first hair growth method which comprises transplanting a composition containing the following components to an incised epidermal site:
(a) dermal papillae or dermal papilla cells; and
(b) epidermal tissue or epidermal cells.

Also disclosed herein is a second hair growth method which comprises transplanting a composition containing the following components to an incised epidermal site:
(a) dermal papillae or dermal papilla cells; and
(c) tissue which constitutes hair follicles or cells thereof.

Also disclosed herein is a third hair growth method which comprises transplanting a composition containing the following components to an incised epidermal site:
(a) dermal papillae or dermal papilla cells;
(b) epidermal tissue or epidermal cells; and
(c) tissue which constitutes hair follicles or cells thereof.

In the aforementioned methods, it is preferred that the incised epidermal site is formed by incision of a part of dermis and whole epidermal layer.

It is also preferred that the components (a), (b) and (c) in each of the aforementioned methods are derived from human or derived from human scalp and that, when the components are derived from human scalp, the aforementioned incised epidermal site is formed in human scalp.

It is also preferred that, in the aforementioned first to third methods, the dermal papilla cells of the component (a) are cultured dermal papilla cells.

In the aforementioned second or third hair growth method, it is preferred that the component (c) is dermal sheath or dermal sheath cells or the dermal papilla cells of the component (a) are cells subcultured for 10 or more passages and the component (c) is dermal sheath or dermal sheath cells of the hair bulb. In the case of such a method, it is preferred that the dermal sheath cells of the component (c) are cultured cells and, more specifically, the dermal sheath cells of the component (c) are cultured cells that are grown in a medium containing FGF 2.

Further, in this application, the following is described as the transplantation material used in each of the aforementioned methods. Thus, a first composition is described containing the following components:
(a) dermal papillae or dermal papilla cells; and
(b) epidermal tissue or epidermal cells.

Also disclosed is a second composition containing the following components:
(a) dermal papillae or dermal papilla cells; and
(c) tissue which constitutes hair follicles or cells thereof.

A third composition is also disclosed containing the following components:
(a) dermal papillae or dermal papilla cells;
(b) epidermal tissue or epidermal cells; and
(c) tissue which constitutes hair follicles or cells thereof.

In those first to third compositions, it is preferred that the components (a), (b) and (c) are derived from human or derived from human scalp.

Further, in the aforementioned first to third composition, it is preferred that the dermal papilla cells of the component (a) are cultured cells.

Still further, in the aforementioned second or third composition, it is preferred that the component (c) is dermal sheath or dermal sheath cells or the dermal papilla cells of the component (a) are cells subcultured for 10 or more passages and the component (c) is dermal sheath or dermal hair cells of the hair bulb. In the case of such a composition, it is preferred that the dermal sheath cells of the component (c) are cultured cells and, more specifically, the dermal sheath cells of the component (c) are cultured cells that are grown in a medium containing FGF 2.

Compositions of the invention are as set out in claims 1 to 6. Uses of the invention are as set out in claims 7 to 12.

In this invention, "dermal papilla" means a dermal papilla tissue which is isolated from the skin and "dermal papilla cell" means each of the cells which constitute the dermal papilla. Similarly, "epidermal tissue" is a tissue of epidermis isolated from the skin and "epidermal cell" is each of the cells which constitute the epidermal tissue. "Dermal sheath" is a dermal sheath itself that is isolated from hair follicle and "dermal sheath cell" means each of the cells constituting the dermal sheath.

"Hair growth" in this invention means that hair follicle is induced into the epidermal cells by transplanted dermal papilla or dermal papilla cells and hair shaft is spontaneously generated from the follicle. Although "hair growth" means that the hair that is generated from the hair follicle as mentioned above spontaneously elongates, the elongation of the hair as such may also be mentioned as "hair growth induction".

Other terms and concepts of the inventions will be illustrated in detail in the description for the mode of carrying out the invention and for Examples. Except the special arts which are clearly mentioned for its source, various arts which are used for carrying out the inventions are able to be easily and surely carried out by persons skilled in the art based on the descriptions in the known documents, etc.

### Brief Description of the Drawings

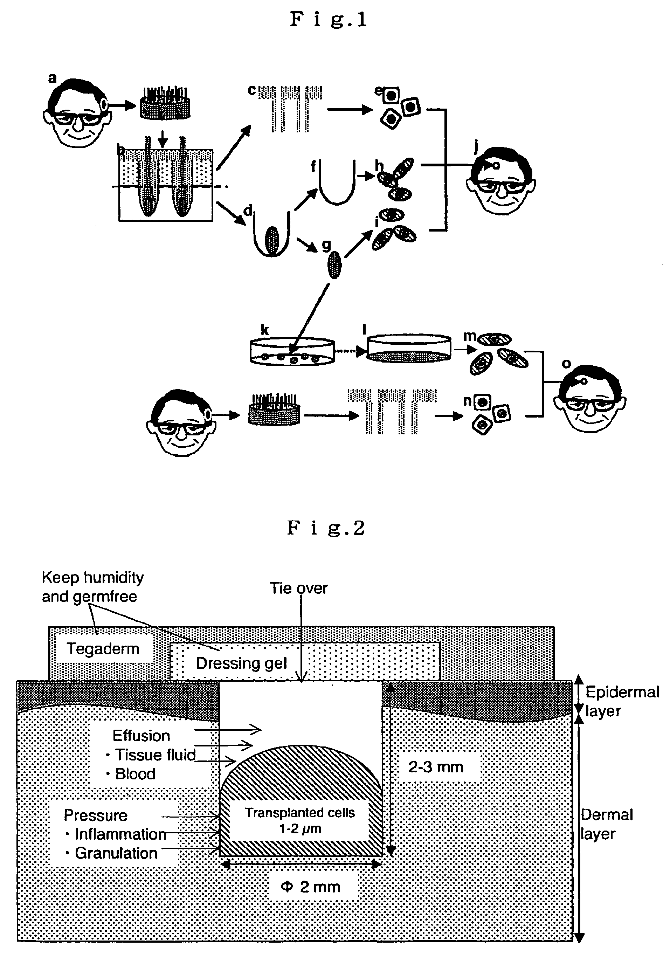

Fig. 1 is a scheme for a mixed transplantation of hair dermal papilla cells and epidermal cells. A haired skin (b) was separated from the back of the head (a) of healthy male. The skin was separated into epidermis (c) and hair bulb (d) by an enzymatic treatment and by handwork using tweezers. The epidermis was made into epidermal cells (e) by a treatment with trypsin while the hair bulb was further separated into dermal hair root sheath (f) and hair dermal papilla (g). The hair dermal papilla and the dermal hair root sheath were subjected to a treatment with collagenase and trypsin to give single cells and labeled with a fluorescent dye DiI to give DiI-labeled hair dermal papilla cells (i) and dermal hair root sheath cells (h). A part of the hair dermal papilla cells were sown on a plastic dish to conduct a primary culture (k). The primarily cultured hair dermal papilla cells were subjected to subcultures for three times (l) and further subjected to labeling with a fluorescent dye to give cultured hair dermal papilla cells (m). Non-cultured epidermal cells (e), dermal hair root sheath cells (h) and hair dermal papilla cells (i) were mixed and subjected to an autologous cell transplantation to the forehead skin wound to which the transplantation is to be done. In the meanwhile, the cultured hair dermal papilla cells (m) were mixed with the epidermal cells (n) which were freshly prepared by the aforementioned method and similarly subjected to an autologous cell transplantation to the forehead skin wound to which the transplantation is to be done (o).
Fig. 2 is a cross-sectional scheme of the transplantation site to which a mixture of dermal papilla cells and epidermal cells is transplanted and of a cover after the operation. A forehead skin under the hair-line where no hair was present was previously selected as the site to which the transplantation is to be done and then all epidermal layer and most of dermal layer of these areas were trepaned to a depth of 3 mm. The mixed cell pellet was injected thereinto.

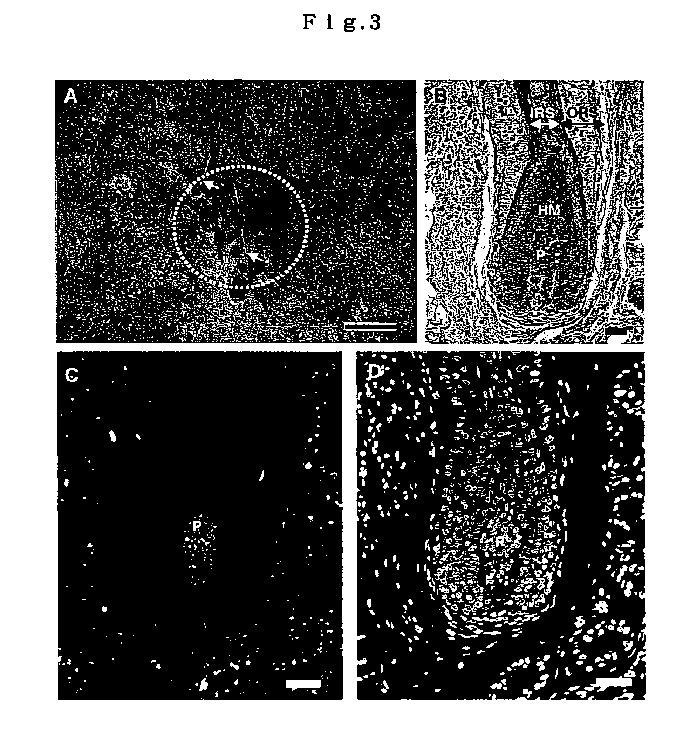

Fig. 3 shows the result clinical study of a cell-mixture transplantation of the freshly prepared dermal papilla cells, the dermal sheath cells and the epidermal cells. A is hairs (an arrow) which were grown after three weeks from the transplantation and the transplanted site (a circle with a broken line). B is a histology of hair follicular bulb of the hair grown at the transplanted site stained with hematoxylin and eosin. P, dermal papilla; HM, hair matrix; IRS, inner root sheath; ORS, outer root sheath. C is a DiI fluorescent photograph on serial section of B. Fluorescent signals are noted at the position P. D is a fluorescent picture of C which is similarly stained at nucleus.

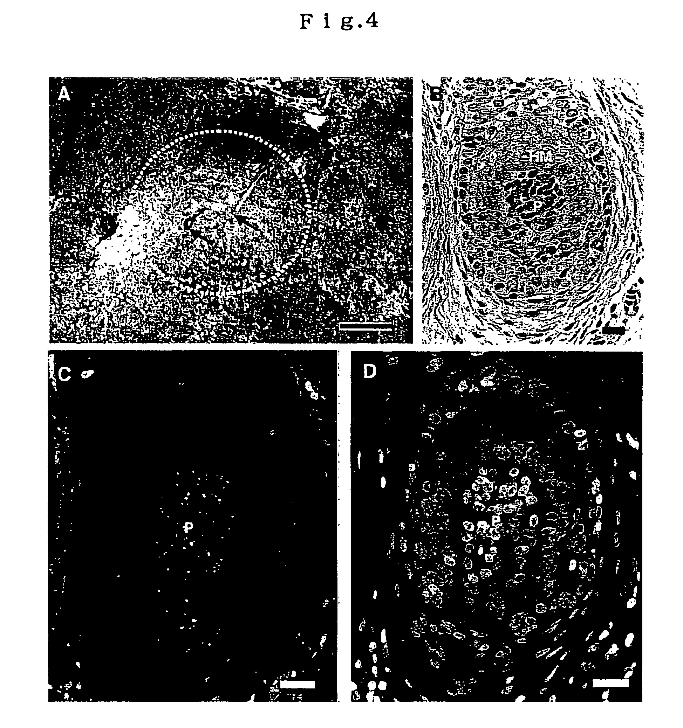

Fig. 4 shows the result of transplantation of a mixture of the subcultured dermal papilla cells and non-cultured epidermal cells in clinical study. A is hair shafts (arrows) which were grown after three weeks from the transplantation and the transplanted site (a circle with a broken line). B is histology of follicular bulb of the hair grown at the transplanted site stained with hematoxylin and eosin. P is dermal papilla and HM is hair matrix. C is a DiI fluorescent microscopy of serial section of B. Fluorescent signals are noted at the position P. D is a fluorescent microscopy of nucleus staining of C.

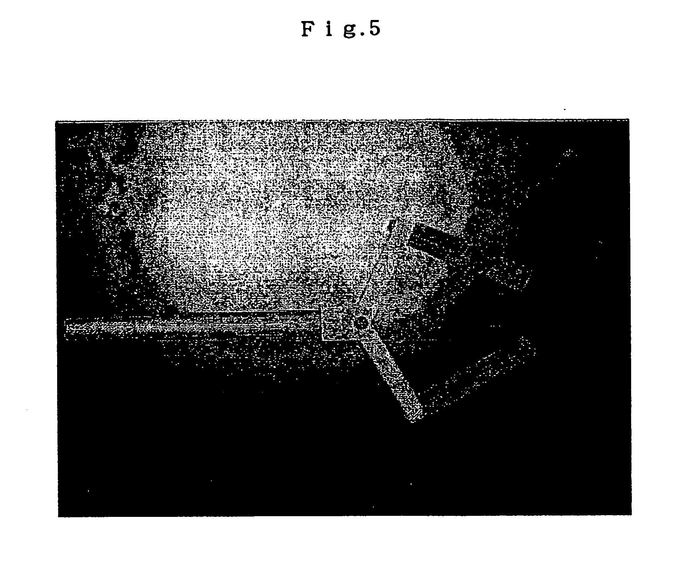

Fig. 5 is a picture of a haft of knife where angle of blade of the knife is able to be freely adjusted. When this device is used, it is possible to form not only the circular incision as Fig. 2 but also the epidermal incision on the line.

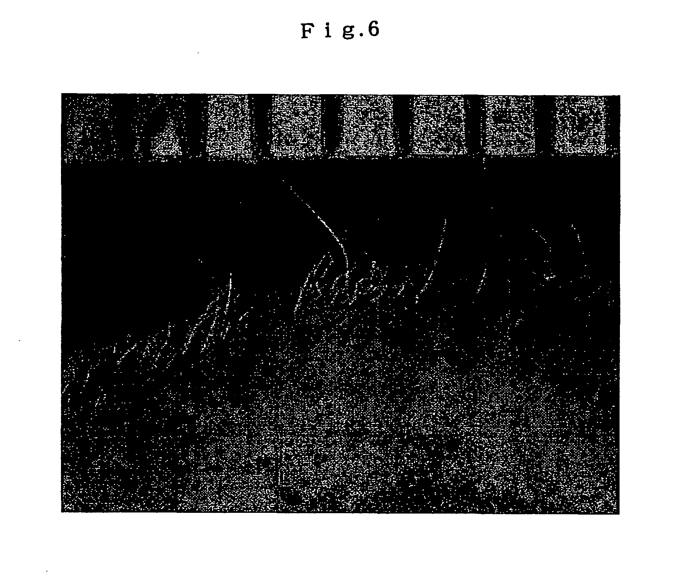

Fig. 6 is a macroscopy of the state of hair growth by heterologous transplantation of a rat cell-mixture of epidermal cells and dermis-derived cells containing dermal papilla cells to nude mouse. In the third week from the transplantation, hair growth aligned on the linear line was achieved. As a result, regeneration of flumina polorum is now possible.
Fig. 7 is a microscopy of a rat vibrissa hair follicle stained with H&E. Dermal sheath is connected to dermal papilla at the lowermost end of the hair bulb and surrounds the hair follicles (heads of arrows). DP, dermal papilla; DS, dermal sheath; M, hair matrix; O, outer root sheath; I, inner root sheath; C, cortex.

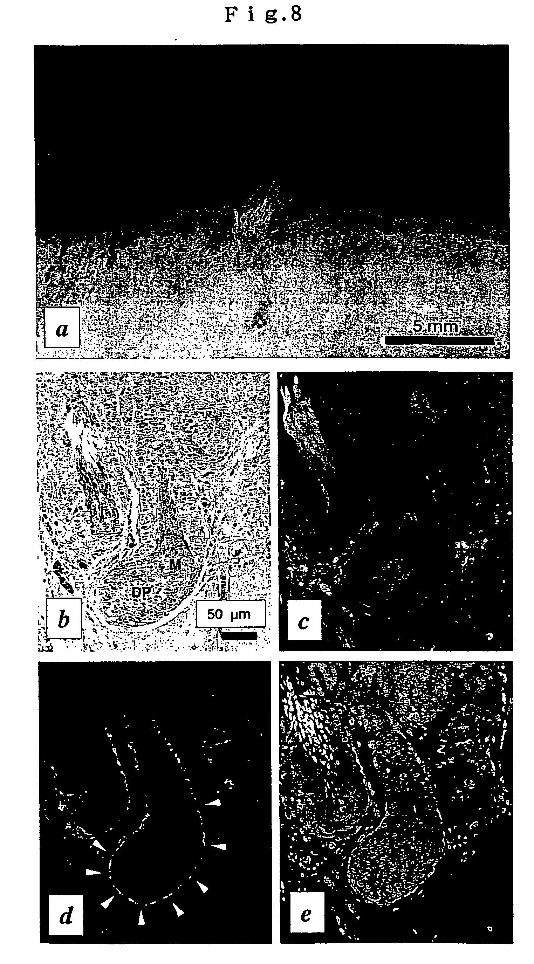

Fig. 8 shows histochemistry of hair growth inducing ability of lowly subcultured dermal papilla cells at 6 passages (*p*=6) and tissues induced thereby. Serially subultured dermal papilla cells (*p*=6) derived from rat whiskers were mixed with freshly prepared epidermal cells of newborn rat and transplanted by a graft chamber method onto the back of nude mouse. After three weeks from the transplantation, hair growth was observed (a: shown by arrows). Paraffin sections of these hair growth tissue were prepared and microscopic observed after staining with HE (b), fluorescent dye DiI (c) and nuclear-staining fluorescent dye Hoechst (e). The serial section was subjected to immunostaining with anti-SM-α-actin antibodies which specifically recognizes dermal sheath layer (d). Positive reaction of the SM-α-actin antibodies was confirmed in the dermal sheath in d, shown by arrow heads. DP is dermal papilla, DS is dermal sheath and M is hair matrix.

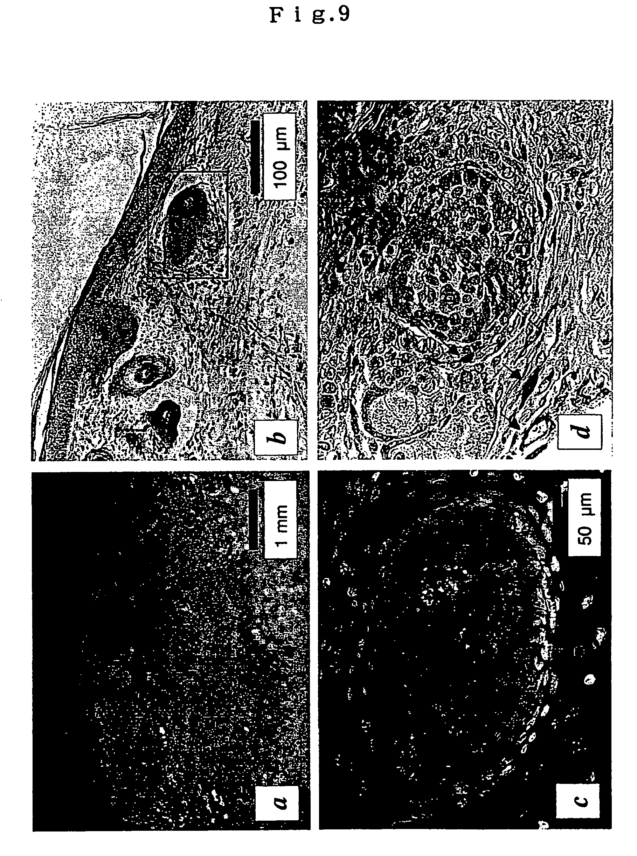

Fig. 9 shows histochemistry of hair growth inducing ability of highly subcultured dermal papilla cells (39 passages) and tissues induced thereby. The highly subcultured dermal papilla cells (n = 39) were mixed with freshly prepared epidermal cells of newborn rat and transplanted by a graft chamber method onto the back of nude mouse. After three weeks from the transplantation, no hair growth was observed (a: shown by broken lines). Paraffin sections were prepared from the site to which cells were transplanted and observed under a microscope after staining with H&E (b) and co-staining with fluorescent dye DiI and nuclear-staining fluorescent dye Hoechst (c). Many Dil-positive cells were observed in dermal papillae (c: in a site with broken line; representative examples are shown by small arrows). The continued slice was subjected to immunostaining with an SM-α-actin antibody which specifically recognizes dermal sheath (d). However, no positive cells were found except in capillary vessels (d: shown by arrows). DP is dermal papilla and M is hair matrix.

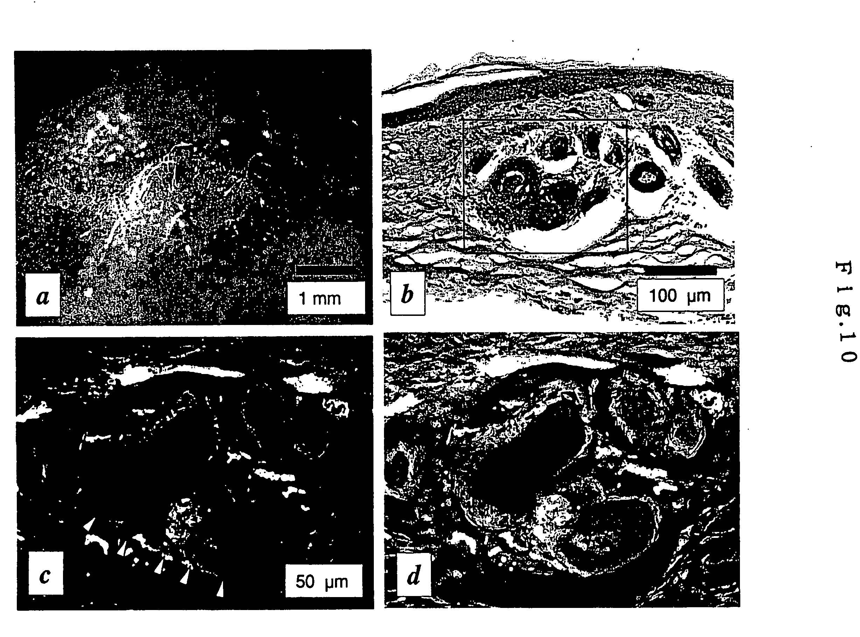

Fig. 10 shows histochemistry of a hair growth inducing ability of cultured dermal sheath cells at passage 1 (*p*=1) and tissues induced thereby. The cultured dermal sheath cells (*p*=1; derived from GFP-Transgenic rat) were mixed with freshly prepared epidermal cells of newborn rat and transplanted by a graft chamber method onto the back of nude mouse. After three weeks from the transplantation, some hair growth was observed (a: shown by broken lines). Frozen sections were prepared from the hair growth site and observed under a microscope after staining with H&E (b) and co-staining with fluorescent dye GFP (c) and nuclear-staining fluorescent dye Hoechst (d). Fluorescence of GFP was observed in a hair bulb part which was formed by induction (c: shown by arrow heads). DP is dermal papilla, DS is dermal sheath and M is hair matrix.

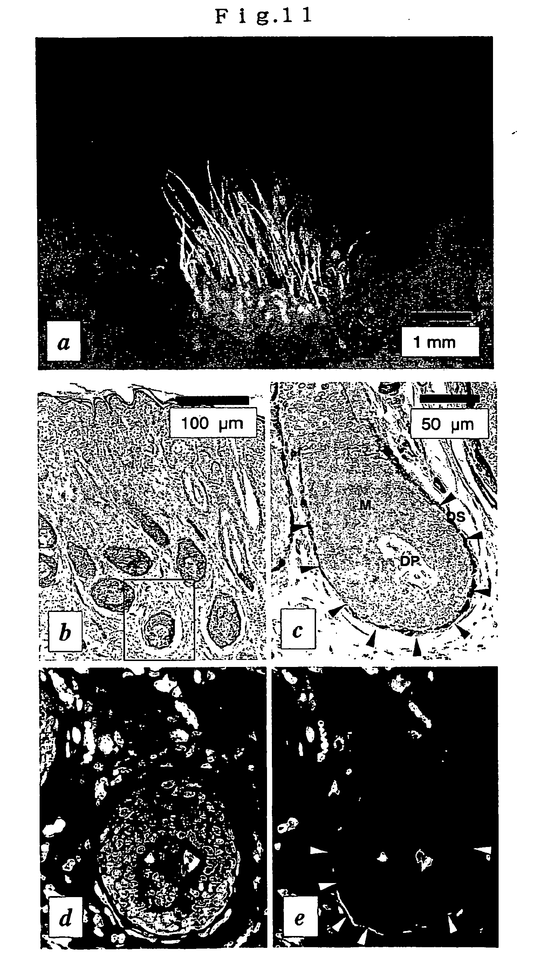

Fig. 11 shows histochemistry of recovery and enhancement of hair growth ability by transplantation of a mixture of highly subcultured dermal papilla cells (*p*=39) and cultured dermal sheath cells (*p*=1) and tissues induced thereby. The highly subcultured dermal papilla cells (*p*=39) and the cultured dermal sheath cells (*p*=1, derived from GFP rat) were mixed with freshly prepared epidermal cells of newborn rat and transplanted by a graft chamber method onto the back of nude mouse. After three weeks from the transplantation, very active hair growth was observed (a: shown by arrows). Frozen slices were prepared from the site to which the cells were transplanted and observed under a microscope after staining with HE (b) and fluorescent dye GFP (e) and co-staining with fluorescent dye DiI and nuclear-staining fluorescent dye Hoechst (d). Many DiI-positive cells were observed in the dermal papillae (d: in the sites of broken lines). Fluorescence of GFP was observed in the dermal sheath of hair bulb part which was subjected to induction and formation (e: shown by arrow heads). The continued slice was subjected to immunostaining with an SM-α-actin antibody which specifically recognizes dermal sheath (c). Positive reaction of the SM-α-actin antibody was confirmed in the dermal sheath (c: shown by arrow heads). DP is dermal papilla, DS is dermal sheath and M is hair matrix.

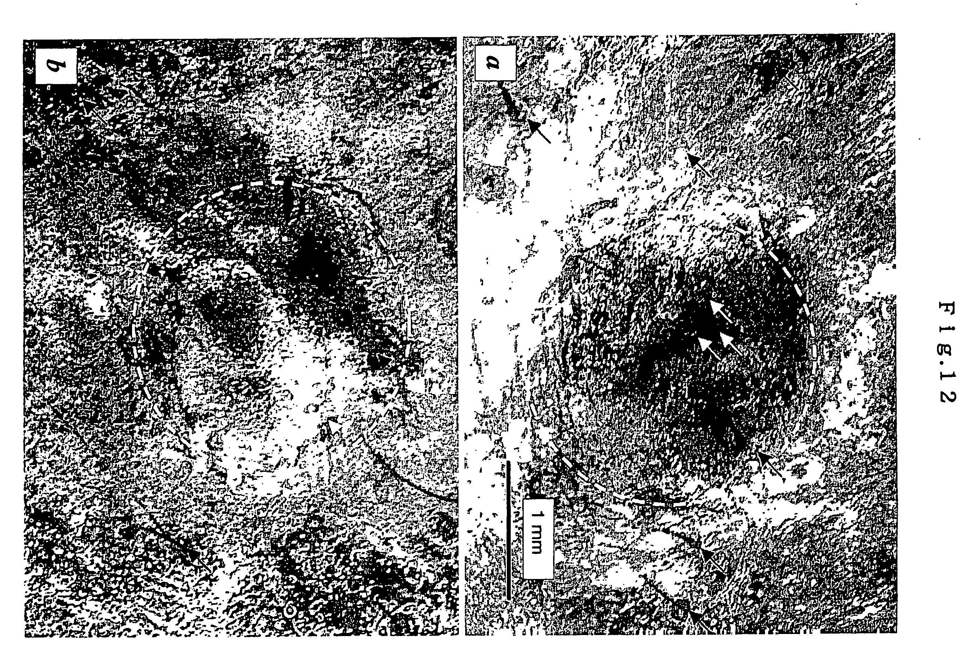

Fig. 12 shows the result of transplantation of a mixture of cultured whisker papilla cells and freshly prepared derma sheath cells to human scalp. Subcultured human dermal papilla cells (*p*=3) and freshly prepared cultured dermal sheath cells were mixed with epidermal cells and subjected to an autologous transplantation to the hairless site of forehead of a volunteer (healthy male, 33-years-old). After two weeks from the transplantation, three black hairs were observed at the site to which a transplantation mixed with dermal sheath cells was conducted (a: in white broken line; white arrows). However, at the site where a transplantation without mixing with dermal sheath cells, only one fine and white hair was observed even after three weeks from the transplantation (b: in white broken line; a white arrow). Black arrows show shafts and pores which were present before the transplantation already.

### Best Mode for Carrying Out the Invention

A first hair growth method is disclosed and is characterized in that a composition (the first composition described above) containing:
(a) dermal papillae or dermal papilla cells; and
(b) epidermal tissue or epidermal cells
is transplanted to an incised epidermal site.

Dermal papillae are able to be isolated using minute tweezers or the like from hair follicles excised from, for example, the skin of animal (such as human scalp). With regard to dermal papilla cells, those which are prepared by dispersing the excised dermal papillae into each cell using collagenase or trypsin or those which are prepared by incubation in an appropriate medium for animal cells (such as an FGF2-added 10% fetal bovine serum-containing Dulbecco-modified Eagle's medium (DMEM 10) mentioned in the Examples) for an appropriate period followed, if necessary, by subjecting to growth by means of subculture for several to several tens of passages may be used. Incidentally, it is preferred that the cultured cells are not in a state of a floated liquid but in such a state that the culture liquid, etc. are removed as much as possible therefrom.

With regard to the epidermal tissue and epidermal cells, it is preferred to use epidermis of the same individual as that wherefrom the dermal papillae are excised and it is more preferred to use epidermal tissue or cells thereof from the skin which is/are as close as possible to the site where the excised dermal papillae were present. For example, epidermal tissue or dispersed cells thereof being adhered to hair follicles excised for isolation of the dermal papillae is/are used.

A mixing ratio of the components (a) to (b) in the composition for the transplantation is able to be freely varied between about 1:9 and about 9:1. The composition may further contain other skin cells (such as fibroblasts of the skin of foot sole). A mixing ratio of the component (a) to the component (b) and the fibroblasts, etc. may also be made from about 1:9 to 9:1.

The epidermis to which the composition for transplantation is to be transplanted may be formed by excising the whole layer of epidermis or a part of dermis with a knife or the like. For example, it is an incision of about 1 to 5 mm length and 1 to 5 mm depth. It is preferred that the injecting amount of the composition to this incised epidermis site is made not more than 10 µl per incision. Cell numbers in that case are not more than about 10⁷ to 10⁸.

A second hair growth method is disclosed which is characterized in that a composition (the second composition described above) containing:
(a) dermal papillae or dermal papilla cells; and
(c) tissue which constitutes hair follicles or cells thereof
is transplanted to an incised epidermal site.

The component (a) of the second composition described above used in this method is the same as the component (a) in the first composition. On the other hand, the composition (c) is follicle-constituting dermal sheath, outer hair root sheath, inner hair root sheath, dermal papilla haft, etc. and dermal sheath or cells thereof are particularly preferred. With regard to the dermal sheath as the component (c), that which is derived from an individual wherefrom the dermal papillae are isolated is preferred and, more preferably, that which is separated from hair follicles excised for isolation of dermal papillae is used. The dermal sheath as it is may be made into a composition by mixing with dermal papillae or dermal papilla cells of the component (a). Alternatively, a product after dispersing it into cells and incubating in an appropriate medium for animal cells may be used. When FGF 2 is added to the medium at that time, the hair root sheath cells are able to be efficiently grown.

A mixing ratio of the components (a) to (c) in the composition for transplantation may be freely varied between about 1:0.1 and 1:0.01. The composition may also contain other skin cells (such as fibroblasts of the skin of the foot sole).

A third hair growth method is disclosed which is characterized in that a composition (the third composition described above) containing the following components:
(a) dermal papillae or dermal papilla cells;
(b) epidermal tissue or epidermal cells; and
(c) tissue which constitutes hair follicles or cells thereof is transplanted to an incised epidermal site.

The components (a) and (b) in the third method are the same as those in the composition of the first method and the component (c) is the same as that in the composition of the second method. Each mixing ratio of the components is also the same as that in the first and the second methods and (the component (a)):(the component (b)) is from about 1:9 to about 9:1 while (the component (a)):(the component (c)) is from about 1:0.1 to about 1:0.01.

When dermal papilla cells where hair growth inducing ability is eliminated or reduced by a long-term subculture (about 10 passages or more in the conventional method or about 15 passages or more in the method of Patent Document 3 by the inventors of this application) are used as the component (a) in the methods of the second and the third methods, it is preferred to use dermal papilla cells or dermal papillae of hair follicle/hair bulb part as the component (c) for achieving an excellent hair growth induction effect.

Compositions according to claims 1 to 6 and uses according to claims 7 to 12 are provided herein.

### Advantages of the Invention

According to the first method, dermal papillae or dermal papilla cells are transplanted together with epidermal tissue or epidermal cells and, as a result, the position and the distance by which the epidermal cells and dermal papilla cells are able to exert an interaction each other are autonomously formed. Epidermal cells do not form a cyst (pathologic solid of epidermis) as well. As a result, reconstitution of the skin and formation of the follicles are achieved in the inner area of the transplanted layer and hair growth from the transplanted dermal papilla cells is promoted.

According to the second method, the component (c) is transplanted together with dermal papillae or dermal papilla cells whereby hair growth induction is promoted and growth of the hair generated from hair follicles is significantly promoted. As a result, flumina pilorum is formed at the hair growing site and, as compared with the skin transplantation and follicle transplantation, more natural reproduced hair is generated.

According to the third method, the components (b) and (c) are transplanted together with dermal papillae or dermal papilla cells whereby follicle formation and hair generation are promoted by the action of the component (b) and, further, elongation of the produced hair is promoted by the action of the component (c). As a result, far more effective reproduction of transplanted hair is now possible as compared with the conventional methods.

### Examples

This invention will be illustrated in more detail and specifically by way of the following Examples although the present invention is not limited by those Examples but is defined by the claims appended to this description.

### Example 1

### 1. Method

### 1-1. Preparation of human cells for transplantation

Skin was collected from the hair-growing area of the back of the head of healthy male volunteers of 32 and 44 years age and subjected to autologous cell transplantation to the hairless area of the forehead. The cells used for the transplantation were separated from the site as shown by Fig. 1 and used after making into single cells by an enzymatic treatment. Details of the preparation of the cells will be mentioned as hereunder.

On the day of 4 weeks before the cell transplantation, scalp of 3 cm² containing hair bulb was collected from the back of the head. The skin material for the operation was separated into the skin and subcutaneous tissue using a microknife. The skin tissue was treated at 37°C for 1 hour with a 10% autoserum DMEM containing 2,000 units/ml dispase to separate into epidermis and dermis. Dermal tissue was finely cut into 1 mm squares, sown on a 3.5-cm plastic dish and subjected to a primary culture and to subcultures for three times according to a usual method. From the subcutaneous tissue, 300 dermal papillae and dermal sheathes were separated. Among them, 10 normal dermal papillae were sown on a 3.5-cm plastic dish and subjected to a primary culture and to subcultures for three times according to the method of Patent Document 2.

On the day of the cell transplantation, scalp of 3 cm² containing hair bulb part was collected once again from the back of the head and separated into the skin and subcutaneous tissue. From the subcutaneous tissue, hair follicles were separated and hair bulb part was separated therefrom. Dermal papillae were separated from the hair bulb part, digested with 0.35% collagenase and 0.25% trypsin EDTA at 37°C for 1 hour and the resulting single cells were stored in a 10% autoserum DMEM of 4°C until use. The skin tissue was treated with a 10% autoserum DMEM containing 2,000 units/ml dispase at 37°C for 1 hour and epidermis containing hair follicles was separated from dermis. The epidermis containing hair follicles was digested with 0.25% trypsin EDTA at 37°C for 1 hour to give single cells. Dermal papilla cells and dermal sheath cells were subjected to a fluorescent staining using DiI. Incidentally, all processes were aseptically conducted on a clear bench or in an aseptic instrument.

### 1-2. Preparation of epidermal and dermal incision sites and cell transplantation

Tattoo was applied on the position of the hairless sites of forehead to which cells were transplanted with a dye where fine particles of carbon were dissolved in 70% ethanol. Pictures with high resolving power of this site were previously taken under a digital microscope (manufactured by Keyence) to record the distribution of hair of the body. After anesthetizing with 1% lidocaine and 1% epinephrine, the skin was excised to the depth of 3 mm using a Trepan (manufactured by Kai) having a diameter of 2.5 mm to prepare a bed for the transplantation (Fig. 2). Freshly prepared dermal papilla cells or cultured dermal papilla cells, epidermal cells, fibroblasts and dermal sheath cells were mixed as shown in Table 1 and the mixed cells were centrifuged at 2,000 rpm for 5 minutes. Since it is necessary to transplant the cells in a state as concentrated as possible, transplantation to the incised skin was conducted by a method where hyaluronic acid gel is layered during centrifugation and, after the centrifugation as such, the cells were extruded using a micro-syringe (Patent Document 3). As shown in Fig. 2, the transplanted site was covered with tagadam and nujel. After the transplantation of the mixed cells to the transplanting bed, they were observed under a digital microscope for the proceeding until 28 days thereafter and the transplanted site was subjected to a biopsy. The biopsy specimen was fixed with 20% formalin for one night, subjected to a paraffin embedding by a conventional method, made into slices of 5 µm thickness and used for a histological observation.

**Table 1**

| Cells | Cell Numbers | |
|---|---|---|
| | Transplantation Example 1 | Transplantation Example 2 |
| Dermal papilla Cells | 2.8 × 10⁴ | 0 |
| Cultured Dermal papilla Cells | 0 | 2.0 × 105 |
| Dermal sheath Cells | 7.0 × 105 | 0 |
| Epidermal Cells | 1.0 × 10⁵ | 2.0 × 105 |
| Volume of Transplanted Cells (unit: µl) | 2.1 | 2.1 |

### 2. Results

Transplantation Example 1: In the autologous transplantation of human dermal papilla cells to forehead (Transplantation Example 1), the transplanted site was completely epithelized on the seventh day. After that, observations were conducted on the development every seven days for six weeks. In the Transplantation Example 1, it was observed that, on the third week from the transplantation, two fine white hair sheaths elongated to an extent of 2 mm (Fig. 3A). On the sixth week from the transplantation, the hair-grown site was collected and histologically observed whereupon five hair follicles were observed in the Transplantation Example 1. When those follicles were subjected to a fluorescent observation, a red fluorescent dye previously labeled to dermal papilla cells and dermal sheath cells was observed in the dermal papillae (Fig. 3B, C and D). Although the similar red autologous fluorescence is noted in human skin, it is excited by the G excited ray only and, therefore, it is able to be distinguished from the labeled dye. Incidentally, in this hair-grown site, there was no hair before transplantation of the cells and, in addition, the skin was completely excised to a depth where hair follicles are distributed.

Transplantation Example 2: In order to confirm the follicle-inducing ability and inducing ability for hair growth of the subcultured human dermal papilla cells only to human epidermis, the subcultured dermal papilla cells were mixed with epidermal cells which were newly prepared from the skin of back of the head and transplanted to the forehead.

During the seven days after the transplantation, the development was the same as that in the Transplantation Example 1 and, in the fourth week after the transplantation, two fine white hair shafts being elongated in 0.3 mm and 0.5 mm each were observed (Fig. 4A). In the fourth week after the transplantation, the hair-grown site was collected and histologically observed whereupon four hair follicles were observed in the Transplantation Example 2. When those hair follicles were subjected to a fluorescent microscopy, red fluorescent dye which was previously labeled to papilla cells was observed in the dermal papillae (Fig. 4B, C and D).

### Example 2

### 1. Methods

### 1-1. Preparation of cells for transplantation

Dermal papilla cells having a hair growth inducing ability and epidermal cells having a hair growth differentiating ability were prepared from the skin of newborn Fischer rat two days after birth. The newborn Fischer rat was sacrificed by decapitation and front and back limbs and tail were excised whereby only a trunk part was remained. Skin of the trunk part was exfoliated, sterilized with Isodine and 70% ethanol, washed with a physiological saline solution and stored at 4°C until it is actually used. Subcutaneous tissue adhered to the skin of the newborn was detached by a micro-knife under a stereoscopic microscope in an aseptic environment. Incidentally, all steps thereafter were aseptically carried out on a clean bench or in an aseptic instrument.

The skin tissue was cut in strips each being about 3 mm in width and 10 mm in length and treated at 4°C for one night in dispase dissolved to become 1,000 units/ml in a Dulbecco-modified Eagle's medium containing 10% of fetal bovine serum. The skin tissue treated with dispase was well washed with a physiological saline solution and separated into epidermis and dermis under an aseptic environment.

The epidermis and dermis were finely cut using a surgical knife and treated with a 0.25% trypsin EDTA solution at 37°C for 10 minutes to prepare a suspension where cells were floated.

The dermis was treated with a 0.35% aqueous physiological saline solution at 37°C for 60 minutes to give a cell suspension where cells were floated. Since the cell suspension where the dermal cells are floated contained an epidermal component forming the hair follicles, it was centrifuged at 300 rpm for 2 minutes to separate a floating fraction comprising dermal cells only.

Each cell suspension was aseptically sieved with the mesh sizes of 100 µm and 40 µm and aggregates where plural cells were adhered to each other were removed.

Epidermal cells and dermal cells in the same cell numbers were mixed and centrifuged at 1500 rpm for 5 minutes to prepare cell pellets. The medium was removed from the pellets followed by storing at ice temperature until transplantation.

### 1-2. Device where knife angle is able to be freely adjusted

A spare blade for a surgical knife was used and a haft where the edge of the blade was able to be crossed at any angle between 10° and 150° (Fig. 5) was prepared. As a result of this mechanism, incision angle of the skin is able to be freely set.

### 1-3. Preparation of the incised site of epidermis and cell transplantation

On the back of BALB/C nu/nu mouse (male; 4 weeks age), incised site of epidermis was prepared 0.5 mm in width and 10 mm in length. A 27G injection needle where its front was removed was attached to a 100-µl microsyringe and the cell pellets were sucked into the syringe. The cell pellets in an amount of 10⁴ were extruded from the syringe and transplanted to the incised site of the epidermis. In order to prevent drying, the site to which the cells were transplanted was applied with a cover where a fibrin paste was solidified.

### 1-4. Observation of hair growth by a mixture of dermal papilla cell-containing dermis-derived cells and epidermal cells

Before the transplantation, an observation under a microscope was conducted to confirm whether body hair was present at the site to be transplanted. Every one to two week(s) after the transplantation, observations under a microscope were conducted to check the changes in the transplanted skin.

### 2. Results

On the third day from the transplantation, the fibrin paste fell off under the normal life state of the mouse whereupon the transplanted wound was in a cured state. When the development thereafter was observed, hair growth was noted on the third week (Fig. 6). The hair growth as such was distributed on a line and coincided with the incised site of the epidermis.

### Example 3

### 1. Methods

### 1-1. Isolation and incubation of whisker papillae and dermal sheath of rat

A male Fischer rat six weeks of age was sacrificed by anesthetizing with diethyl ether and the cheek was excised. The excised cheek was sterilized with Isodine (Meiji Seika) and 70% ethanol and washed with a physiological saline solution. Dermal papillae were carefully isolated from the excised hair follicles using a fine tweezers and sown on a 35-mm incubation dish (manufactured by Becton Dickinson). A primary culture was conducted for 2 to 3 weeks on a Dulbecco-modified Eagle's medium containing 10% fetal bovine serum to which FGF 2 was added (DMEM 10) and the medium was exchanged every five days. After the primary culture, subcultures were conducted every seven to ten days. For the transplantation, cells of 6 and 39 passages were used.

Male adult EGFP transgenic Wistar rat (K. K. Wyeth Laboratories) was sacrificed by anesthetizing with diethyl ether and its cheek was excised. The excised cheek was sterilized the same as above and then hair follicles were excised. Dermal sheath was isolated from the excised hair follicles and sown on a 35-mm culture dish. A primary culture was carried out on an FGF2-added DMEM 10 for 2 to 3 weeks and the medium was exchanged every 3 to 4 days. After the primary culture, subcultures were conducted every seven to ten days. For the transplantation, cells of 1 passage were used.

### 1-2. Preparation of epidermis of newborn rat

Dermal papilla cells having a hair growth inducing ability and epidermal cells having a hair growth differentiating ability were prepared from the skin of newborn Fischer rat two days after birth. The newborn Fischer rat was sacrificed by anesthetizing with diethyl ether and front and back limbs and tail were excised whereby only a trunk part was remained. Skin of the trunk part was exfoliated, sterilized with Isodine and 70% ethanol, washed with a physiological saline solution and stored at 4°C until it is actually used. Subcutaneous tissue adhered to the skin of the newborn was detached by a micro-knife under a stereoscopic microscope in an aseptic environment. Incidentally, all steps thereafter were aseptically carried out on a clean bench or in an aseptic instrument.

The skin tissue was cut in stripes each being about 3 mm in width and 10 mm in length and treated at 4°C for one night in dispase (manufactured by Sankyo Junyaku Kogyo) dissolved to become 1,000 units/ml in DMEM 10. The skin tissue treated with dispase was well washed with a physiological saline solution and separated into epidermis and dermis under an aseptic environment.

The epidermis was finely cut using a surgical knife and treated with a 0.25% trypsin EDTA solution at 37°C for 10 minutes to prepare a suspension where cells were floated.

Each cell suspension was passed through filters with the mesh sizes of 100 µm and 40 µm and aggregates where plural cells are adhered to each other were removed.

### 1-3. Preparation of fibroblasts from dermis of sole of adult rat

Male Fischer rat ten weeks of age was sacrificed by anesthetizing with diethyl ether and the skin of the sole was excised. The excised sole skin was sterilized with Isodine and 70% ethanol and washed with a physiological saline solution. The subcutaneous tissue adhered to the sole skin was removed by a micro-knife in an aseptic environment.

After removal, it was divided into four equal parts and treated at 4°C for one night with a dispase solution dissolved to become 1,000 units/ml in DMEM 10. The skin tissue treated with dispase was well washed with a physiological saline solution whereby epidermis and dermis were separated. The dermis was finely cut into squares of 1 to 2 mm and explanted on a 60-mm incubation dish (manufactured by Becton Dickinson) and a primary culture of the fibroblasts was carried out.

After the primary culture, the fibroblasts derived from dermis of adult rat sole were subcultured and the cells up to 2 to 4 passages were used for the transplantation.

### 1-4. Mixed transplantation of rat whisker papilla cells, GFP rat whisker dermal hair sheath cells, newborn rat epidermal cells and fibroblasts derived from adult rat sole dermis

A male nude mouse 4 weeks of age (manufactured by Nippon Charles River) was anesthetized by intraperitoneal administration of Somnopentyl and, sterilized with Isodine and the skin of the whole layer of the flank was excised in a circle of 7 mm diameter. A graft chamber was attached to this site using a suture made of Nylon. Rat whisker papilla cells (6 and 39 passages), GFP rat whisker dermal hair sheath cells (1 passage), newborn rat epidermal cells and fibroblasts derived from adult rat sole dermis which were previously labeled with a fluorescent dye (DiI) were mixed in a ratio as shown in Table 2 and centrifuged at 2000 rpm for 5 minutes to prepare cell pellets. After removal of a medium from the pellets, they were injected into a graft chamber using a micro-pipette. The above operation was aseptically carried out. During one week after the transplantation, the transplanted site was protected with a surgical tape (manufactured by Nichiban). After one week from the cell transplantation, the graft chamber was removed, the transplanted site was disinfected with Isodine and breeding was conducted for further two weeks paying careful attention to onset of infectious diseases.

### 1-5. Hair growth by cell transplantation and observation of the tissue

The transplanted site after 3 weeks from the operation was observed under a stereoscopic microscope (manufactured by Leica) and pictures of the state of hair growth were taken. After taking the pictures, the transplanted site was excised, fixed for one night and day with Mildform 10N and embedded in paraffin. The finely cut slice (5 µm) was subjected to staining with hematoxylin and eosin (HE) and to fluorescent staining of nuclei and confirmation of the fluorescent dye DiI labeled to the papillae and GFP of the rat whisker dermal sheath cells was conducted. There was also carried out an immunostaining of the continued slices using anti-SM-α-actin antibodies which was a marker for dermal sheath.

**Table 2**

| Rat Cells | Cell Numbers | | |
|---|---|---|---|
| | Transplantation Example 1 | Transplantation Example 2 | Transplantation Example 3 |
| Epidermal cells of newborn | 10⁷ | 10⁷ | 10⁷ |
| Whisker papilla cells (6 and 39 passages) | 3 × 106 | | |
| Fibroblasts derived from foot sole dermis | 7 × 10⁶ | 7 × 10⁶ | 4 × 106 |
| Whisker dermis hair root sheath cells (1 passage) | | 3 × 10⁶ | 3 × 106 |
| Whisker papilla cells (39 passages) | | | 3 × 10⁶ |

### 2. Results and discussions

With regard to hair growth ability of the rat whisker papilla cells, analysis using a graft chamber was carried out. High (*p*=39) and low (*p*=6) passaged cultured dermal papilla cells rat whisker (Fig.8, *p*=6 and Fig. 9, *p*=39) were transplanted by mixing with newborn rat epidermal cells and fibroblasts derived from dermis of foot sole of rat. After one week from the cell transplantation, skin which was derived from the transplanted cells was found to be formed. After three weeks from the cell transplantation, hair growth was confirmed in the site to which the cultured whisker papilla cells (*p*=6) were transplanted (Fig. 8a) but no hair growth was observed in the case of using the high passaged dermal papilla cells (Fig. 9a). However, hair follicles were confirmed of both tissues microscopically with H&E staining (Fig. 8b and Fig. 9b) and it was confirmed that, although the high passaged dermal papilla cells (*p*=39) have no hair-shafts growth ability, they have an inducing ability for hair follicles. In the very near serial section, fluorescence of DiI labeling dermal papilla cells previously transplanted was confirmed and it was confirmed to be hair growth or hair follicle by the transplanted dermal papilla cells (Fig. 8c and d and Fig. 9c). In the hair follicles induced by those cells, an immunostaining was carried out using anti-SM-α-actin antibodies which was a marker for dermal sheath in order to check whether dermal sheath was formed. The result was that a positive reaction was observed only in the follicle where hair growth was resulted by the low passaged cells (p=6) whereby formation of dermal sheath was confirmed (arrow heads in Fig. 8d and e) while, in the hair follicles formed by the high passaged cells (p=39), no positive reaction was observed (Fig. 9d). From the above result, it was now made clear that, in the whisker papilla cells of rat, ability for inducing the hair growth decreased and was lost and ability for forming the dermal sheath was also lost as the passage increased.

Next, dermal sheath cells were added to the whisker papilla cells of rat where hair growth inducing ability was lost by subculturing for a long period and analysis was similarly carried out using a graft chamber. In a group where cultured dermal sheath cells (*p*=1) was solely added, hair growth to some extent was observed (Fig. 10a). From the observation of pictures stained with HE (Fig. 10b) and fluorescence (Fig. 10c), fluorescence of GFP was confirmed in the dermal papillae of hair follicles and it was confirmed to be the hair growth by dermal sheath cells. Since fluorescence of GFP was also observed in dermal sheath, it was confirmed to be formed by the transplanted dermal sheath cells (Fig. 10c). On the contrary, in a group where rat whisker papilla cells of rat (39 passages) and cultured dermal sheath cells (1 passage) were transplanted together, an apparently significant hair growth was observed (Fig. 11a). From the observation of stained pictures of the tissue with HE (Fig. 11b) and fluorescence (Fig. 11c), fluorescence of DiI and fluorescence of GFP were confirmed in dermal papillae and dermal sheath, respectively. In addition, as a result of immunostaining using anti-SM-α-actin antibodies, formation of dermal sheath was confirmed in the group where hair growth was done by addition of the cultured dermal hair room sheath cells (Fig. 11e). From the above results, it is now apparent that formation of dermal sheath was important for hair growth and that, when the dermal sheath cells were added to the dermal papilla cells losing a hair growth ability, the hair growth ability recovered. From such a fact, it was suggested that, when dermal sheath cells were added to dermal papilla cells having an inducing ability for hair growth, higher hair growth induction was possible.

### Example 4

### 1. Methods

### 1-1. Preparation of human cells for transplantation

Skin was collected from hair-grown area of back of head of a healthy male volunteer of 33 years age and subjected to an autologous cell transplantation to hairless area in the forehead. Details of the preparation of the cells will be mentioned as follows.

Four weeks before the cell transplantation, scalp of 3 cm² including hair bulb part was collected from the back of the head. The skin as a material for the operation was separated into skin and subcutaneous tissue using a micro-knife. The skin tissue was treated at 37°C for 1 hour with a 10% autoserum DMEM containing 2000 units/ml of dispase to separate into epidermis and dermis. The dermal tissue was finely cut into 1-mm square, sown on a 3.5-cm plastic dish and subjected to a primary culture and subcultures 3 times according to a common method. From the subcutaneous tissue, 300 dermal papillae and dermal sheath were separated. Ten normal dermal papillae among the above were sown on a 3.5-cm plastic dish and, according to the method of Patent Document 2, a primary culture and subcultures were carried out three times.

On the day when the cell transplantation was conducted, scalp of 3 cm² containing hair follicles was collected again and the skin and a subcutaneous tissue were separated. Hair follicles were separated from the subcutaneous tissue and hair bulb part was separated. Dermal papillae were separated from the hair bulb part and digested at 37°C for 1 hour with 0.35% collagenase and 0.25% trypsin EDTA and the resulting single cells were stored in a 10% autoserum DMEM of 4°C until actual use. The skin tissue was treated at 37°C for 1 hour with a 10% autoserum DMEM containing 2000 units/ml of dispase and epidermis containing hair follicles was separated from dermis. The dermis containing hair follicles was digested at 37°C for 1 hour with a 0.25% trypsin EDTA to give single cells. The dermal papilla cells and the dermal sheath cells were subjected to a fluorescent staining with Dil. Incidentally, all steps were aseptically carried out on a clean bench or in an aseptic instrument.

### 1-2. Preparation of incised sites of epidermis and dermis and cell transplantation

Tattoo was applied on the position of the hairless sites of forehead to which cells were transplanted with a dye where fine carbon particles were dissolved in 70% ethanol. Pictures with high resolving power of this site were previously taken under a digital microscope (manufactured by Keyence) to record the distribution of hair of the body. After anesthetizing with 1% lidocaine and 1% epinephrine, the skin was excised to the depth of 3 mm using a Trepan (manufactured by Kai) having a diameter of 2.5 mm to prepare a bed for the transplantation (Fig. 2). Freshly prepared dermal papilla cells or cultured dermal papilla cells, epidermal cells, fibroblast cells and dermal sheath cells were mixed as shown in Table 1 and the mixed cells were centrifuged at 2,000 rpm for 5 minutes. Since it was necessary to transplant the cells in a state as concentrated as possible, transplantation to the incised skin was conducted by a method where hyaluronic acid gel was layered during centrifugation and, after the centrifugation as such, the cells were extruded using a micro-syringe (Patent Document 3). As shown in Fig. 2, the transplanted site was covered with tagadam and nujel. After transplanting to the transplanting bed, the mixed cells were observed under a digital microscope for the proceeding until 28 days thereafter and the transplanted site was subjected to a biopsy. The biopsy specimen was fixed with 20% formalin for one night, subjected to a paraffin embedding by a conventional method, made into slices of 5 µm thickness and used for a histological observation.

**Table 3**

| Human Cells | Cell Numbers | |
|---|---|---|
| | Transplantation Example 1 | Transplantation Example 2 |
| Epidermal Cells | 1.67 × 105 | 1.67 × 105 |
| Papilla Cells (3 passages) | 1.0 × 10⁵ | 1.0 × 10⁵ |
| Dermal sheath Cells | 1.0 × 10⁵ | 0 |
| Fibroblasts | 0 | 1.0 × 105 |
| Volume of Transplanted Cells (unit: µl) | 5.1 | 5.1 |

### 2. Results

In order to confirm the enhancement of hair growing ability by a transplantation of a mixture of the subcultured human dermal papilla cells and the dermal sheath cells, the subcultured dermal papilla cells were mixed with epidermal cells and dermal sheath cells newly prepared from the skin of the back of head and transplanted to the forehead. In addition, in order to make the ratio of epidermal cells to dermal cells 1:1, they were mixed with cultured fibroblasts (*p*=3). The dermal papilla cells (*p*=3) were cultured for 6 days and the average cell increasing time during that period was 40 hours.

The transplanted site was completely epithelized on the third day. After that, observations were conducted on the development every seven days. As a result of the observations on the development after the transplantation, growth of three hairs was observed after two weeks in a group where dermal sheath cells were added (Transplantation Example 1) (Fig. 12a) while, in a group where they were not added (Transplantation Example 2), growth of only one hair was firstly confirmed on the third week (Fig. 12b).

From the above results, it was now made clear that, in the case of transplantation of hair follicles, an apparently high hair growth induction was able to be achieved when dermal sheath cells were added.

## Claims

1. A composition for use in inducing hair growth, containing
(a) human dermal papilla cells;
(b) human epidermal cells; and
(c) human dermal sheath cells,
wherein the cells (a), (b) and (c) are single cells.

2. The composition according to claim 1, wherein the components are derived from human scalp.

3. The composition according to claims 1 or 2, wherein the dermal papilla cells of the component (a) are cultured cells.

4. The composition according to any one of claims 1 to 3, wherein the dermal papilla cells of the component (a) are cells subcultured for 10 or more passages and the component (c) is dermal sheath cells of the hair bulb.

5. The composition according to any one of claims 1 to 4, wherein the dermal sheath cells of the component (c) are cultured cells.

6. The composition according to any one of claims 1 to 5, wherein the dermal sheath cells of the component (c) are cultured cells which are grown in a medium containing FGF2.

7. The use of a composition containing
(a) human dermal papilla cells;
(b) human epidermal cells; and
(c) human dermal sheath cells,
in the manufacture of an agent for use in inducing hair growth, wherein the cells (a), (b) and (c) are single cells.

8. Use according to claim 7, wherein the components are derived from human scalp.

9. Use according to claim 7 or 8, wherein the dermal papilla cells of the component (a) are cultured cells.

10. Use according to any one of claims 7 to 9, wherein the dermal papilla cells of the component (a) are cells subcultured for 10 or more passages and the component (c) is dermal sheath cells of the hair bulb.

11. Use according to any one of claims 7 to 10, wherein the dermal sheath cells of the component (c) are cultured cells.

12. Use according to any one of claims 7 to 11, wherein the dermal sheath cells of the component (c) are cultured cells which are grown in a medium containing FGF2.

## Patentansprüche

1. Zusammensetzung für die Verwendung beim Fördern von Haarwuchs, die enthält
(a) menschliche dermale Papillenzellen;
(b) menschliche epidermische Zellen; und
(c) menschliche dermale Hüll- bzw. Scheidenzellen,
wobei die Zellen (a), (b) und (c) einzelne Zellen sind.

2. Zusammensetzung nach Anspruch 1, wobei die Komponenten von der menschlichen Kopfhaut stammen.

3. Zusammensetzung nach Anspruch 1 oder 2, wobei die dermalen Papillenzellen der Komponente (a) gezüchtete Zellen sind.

4. Zusammensetzung nach einem der Ansprüche 1 bis 3, wobei die dermalen Papillenzellen der Komponente (a) Zellen sind, die für 10 oder mehr Durchgänge subkultiviert sind, und die Komponente (c) dermale Hüllzellen des Haarbulbus sind.

5. Zusammensetzung nach einem der Ansprüche 1 bis 4, wobei die dermalen Hüllzellen der Komponente (c) gezüchtete Zellen sind.

6. Zusammensetzung nach einem der Ansprüche 1 bis 5, wobei die dermalen Hüllzellen der Komponente (c) gezüchtete Zellen sind, die in einem Medium, das FGF2 enthält, gezüchtet wurden.

7. Verwendung einer Zusammensetzung, die enthält
(a) menschliche dermale Papillenzellen;
(b) menschliche epidermische Zellen; und
(c) menschliche dermale Hüllzellen,
in der Herstellung eines Wirkstoffs für die Verwendung beim Hervorrufen von Haarwuchs, wobei die Zellen (a), (b) und (c) einzelne Zellen sind.

8. Verwendung nach Anspruch 7, wobei die Komponenten von der menschlichen Kopfhaut stammen.

9. Verwendung nach Anspruch 7 oder 8, wobei die dermalen Hüllzellen der Komponente (c) gezüchtete Zellen sind.

10. Verwendung nach einem der Ansprüche 7 bis 9, wobei die dermalen Papillenzellen der Komponente (a) Zellen sind, die für 10 oder mehr Durchgänge subkultiviert sind, und die Komponente (c) dermale Hüllzellen des Haarbulbus sind.

11. Verwendung nach einem der Ansprüche 7 bis 10, wobei die dermalen Hüllzellen der Komponente (c) gezüchtete Zellen sind.

12. Verwendung nach einem der Ansprüche 7 bis 11, wobei die dermalen Hüllzellen der Komponente (c) gezüchtete Zellen sind, die in einem Medium, das FGF2 enthält, gezüchtet wurden.

## Revendications

1. Composition à utiliser pour favoriser la croissance des cheveux, contenant :
(a) des cellules de papilles dermiques humaines ;
(b) des cellules épidermiques humaines ; et
(c) des cellules de gaine dermique humaines,
dans laquelle les cellules (a), (b) et (c) sont des cellules uniques.

2. Composition selon la revendication 1, dans laquelle les composants sont dérivés de cuir chevelu humain.

3. Composition selon les revendications 1 ou 2, dans laquelle les cellules de papilles dermiques du composant (a) sont des cellules en culture.

4. Composition selon l'une quelconque des revendications 1 à 3, dans laquelle les cellules de papilles dermiques du composant (a) sont des cellules repiquées pour 10 passages ou plus et le composant (c) est constitué des cellules de gaine dermique du bulbe capillaire.

5. Composition selon l'une quelconque des revendications 1 à 4, dans laquelle les cellules de gaine thermique du composant (c) sont des cellules en culture.

6. Composition selon l'une quelconque des revendications 1 à 5, dans laquelle les cellules de gaine dermique du composant (c) sont des cellules en culture qui sont cultivées dans un milieu contenant du FGF2.

7. Utilisation d'une composition contenant :
(a) des cellules de papilles dermiques humaines ;
(b) des cellules épidermiques humaines ; et
(c) des cellules de gaine dermique humaines,
pour la fabrication d'un agent à utiliser pour favoriser la croissance capillaire, dans laquelle les cellules (a), (b) et (c) sont des cellules uniques.

8. Utilisation selon la revendication 7, dans laquelle les composants sont dérivés d'un cuir chevelu humain.

9. Utilisation selon la revendication 7 ou 8, dans laquelle les cellules de papilles dermiques du composant (a) sont des cellules en culture.

10. Utilisation selon l'une quelconque des revendications 7 à 9, dans laquelle les cellules de papilles dermiques du composant (a) sont des cellules repiquées pour 10 passages ou plus et le composant (c) est constitué des cellules de gaine dermique du bulbe capillaire.

11. Utilisation selon l'une quelconque des revendications 7 à 10, dans laquelle les cellules de gaine dermique du composant (c) sont des cellules en culture.

12. Utilisation selon l'une quelconque des revendications 7 à 11, dans laquelle les cellules de gaine dermique du composant (c) sont des cellules en culture qui sont cultivées dans un milieu contenant du FGF2.
